# EUROPEAN PATENT APPLICATION

(11) **EP 1 559 375 A1**
(43) Date of publication of application: **03.08.2005**
(21) Application number: 04030805.8
(22) Date of filing: 27.12.2004
(51) Int. Cl.: A61B 17/15

(54) **Knee computer-aided navigation instruments**

(30) Priority: 30.01.2004 US 768520
(71) Applicant: Howmedica Osteonics Corp., Mahwah, NJ 07430 (US)
(72) Inventor: Walsh, Gearoid, Ennis Co. Clare (IE); Chauhan, Sandeep K., Putney London, SW18 1QQ (GB)
(74) Representative: Liebetanz, Michael

(57) **Abstract**

A bone cutting guide block has a saw guide surface thereon and has a body shaped to conform to the anterior-medial shape of the proximal left tibia and distal left femur and also to the anterior-lateral shape of the proximal right tibia or the distal right femur. The block is used by placing the curved inner surface adjacent the anterior-medial or anterior-lateral quadrant of either the tibia or the femur and utilizing images from a computer database utilizing an optical tracking system mounted on the cutting block and referencing to the bone to be cut. Utilizing the tracking system, the cutting guide is positioned preferably by hand in the proximal-distal direction to set the depth of the resection. The cutting guide surface is, then oriented by hand to the correct varus-valgus and/or flexion-extension angles and pinned to the bone. Preferably, the block is pinned using a single pin in either the correct varus-valgus or flexion-extension position and then the other of the angles not initially selected is set by rotating the block about the single pin. Once the other of the varus-valgus or flexion-extension angles is determined, additional pins are utilized to fix the guide in position for use during the resection of the proximal tibia or distal femur. A second block is provided conforming to the anterior-lateral left tibia and femur as well as the anterior-medial shape of the right tibia and femur.

## Description

### BACKGROUND OF THE INVENTION

Total knee arthroplasty involves replacement of portions of the patella, femur and tibia with artificial components. In particular, a proximal portion of the tibia and a distal portion of the femur are cut away (resected) and replaced with artificial components. In performing this knee surgery, it has been desirable to minimize the size of the incision to thereby minimize damage to soft tissue.

In particular, it is necessary to resect the proximal tibia and distal femur with, among other cuts, a facing pair of planer cuts from which other bone cuts, in the case of the femur, can be referenced.

In order to make these cuts, resecting the femur and tibia necessitated significant cutting of soft tissue, including muscles, tendons and ligaments.

As used herein, when referring to bones or other parts of the body, the term "proximal" means closer to the heart and the term "distal" means more distant from the heart.

There are several types of knee prosthesis known in the art. One type is sometimes referred to as a "resurfacing type." In these prostheses, the articular surface of the distal femur and the proximal tibia are "resurfaced" with respective metal and plastic condylar-type articular bearing components. Preferably, these components are made of titanium alloy or a cobalt-chrome alloy such as VITALLIUM® alloy.

One important aspect of these procedures is the correct resection of the distal femur and proximal tibia. These resections must provide planes which are correctly angled in order to properly accept and align the prosthetic components. In particular, the resection planes on the tibia and femur must be correctly located relative to at least three parameters: proximal-distal location; varus-valgus angle; and flexion-extension angle.

Moreover, following distal resection, the femur must be further shaped with the aid of a cutting block, the cutting block must be correctly located relative to internal-external rotation, medial-lateral position and anterior-posterior position. Recently, various computerized systems have been introduced to aid the practitioner during 'different surgical procedures. These systems improve accuracy of the cuts based on accurately locating the mechanical axis of the tibia and femur and provides the ability to verify the cuts made. In these systems, there is utilized multiple video cameras which are deployed above the surgical site and a plurality of dynamic reference frame devices also known as trackers. These trackers are attached to body parts and the surgical instruments and preferably include light emitting devices, such as light emitting diodes, which are visible to the video cameras. Using software designed for a particular surgical procedure, a computer receiving input from the camera guides the placements of the surgical instruments with respect to landmarks on the body. Such systems are shown in U.S. Patent Nos. 6,385,475 and 6,514,259, the teachings of which are incorporated herein by reference. The instrumentation of the present invention includes components for resecting the distal femur and proximal tibia with alignment system for properly positioning the cutting guide surfaces utilizing an optical tracking system optically coupled to a computer system. The design of the present invention allows the resection of the proximal end of the tibia and the distal end of the femur to be' performed anteriorly-medially or anteriorly-laterally as contrasted with the typical resection systems of the prior art in which the resections are all performed from the anterior direction.

U.S. Patent Publication No. 2003/0171757 relates to an instrument that permits resection of the proximal end of the tibia and distal end of the femur to be performed either medially or laterally.

### SUMMARY OF THE INVENTION

The invention relates to a method of forming the distal planer resection of the femur and the proximal planer resection of the tibia utilizing optical tracking systems and computer-aided surgery. The cutting block includes a moveable tracker thereon. The tracker preferably includes at least three light emitting diodes so that the plane where the cut to be made is mounted can be determined by the computer system via video camera inputs. Use of the tracker allows the surgeon to orient the cutting block free-handedly adjacent the bone of either the tibia or the femur and align the cutting surface in the correct proximal-distal direction and at least one of the flexion-extension or varus-valgus angles. By free-hand it is meant that without extramedullary alignment or intramedullary alignment. Once the proper positioning has occurred, the surgeon then inserts a single pin through the cutting block into the femur or the tibia. The surgeon then uses the axis of the pin as a pivoting point for the rotation of the cutting block in the other of the flexion-extension or varus-valgus angles. Once the proper planer position is achieved by the rotation about the pivot pin, at least one additional pivot pin is placed into the block to lock the orientation thereof in a fixed position.

These and other objects are achieved by a method for aligning the varus-valgus, proximal-distal and flexion-extension orientation of a bone cut for use in total knee arthroplasty. Initially, the inner surface of a cutting block having a cutting guide surface thereon is placed adjacent the end of the long bone such as the proximal tibia or distal femur. The orientation of the cutting guide surface is then set in the proximal-distal direction and one of said varus-valgus and flexion-extension angular orientations prior to attaching said cutting block to the bone.

The block is then pinned to the bone and then the block is pivoted around the single pin to allow the cutting guide surface in the other of the varus-valgus or flexion-extension orientations. The oriented block is then pinned with at least one additional pin. The method can be performed free-hand by the surgeon because the cutting block includes a tracking device mounted thereon communicating with a computer preferably using optical outputs on the tracking device and optical inputs to the computer. The computer system can display an image on a display device for guiding the surgeon in the free-hand placement of the cutting block adjacent the long bone.

The cutting block has an inner surface generally conforming to at least a part of the anterior and medial or lateral bone end surfaces such as the end surface of the distal femur or proximal tibia. Preferably, the inner surface conforms to the anterior-lateral quadrant of the bone or the anterior-medial quadrant of the bone. Generally, this results in a curved inner surface which curve may be undulating to match typical anatomy. The block includes a plurality of through holes axially aligned with the plane of the cutting surface. The holes receive a plurality of pins which fix the block to the bone so that when the block is pinned at a predetermined angle, the cutting guide surface is oriented in the same angular orientation.

### BRIEF DESCRIPTION OF THE DRAWINGS

FIG. 1 is an isometric view of the distal femur with a cutting block of the present invention mounted thereon with a plurality of bone pins;
FIG. 2 is an isometric bone cutting block shown in FIG. 1 mounted on the anterior-medial quadrant of the tibia;
FIG. 3 is a front isometric view of the cutting block shown in FIGS. 1 and 2;
FIG. 4 is a side elevation view of the cutting block shown in FIGS. 1-3;
FIG. 4a shows the kit of the present invention having four cutting blocks, two standard and two extended, which are all similar to the block of FIGS. 1 to 4;
FIG. 5 is a rear view of the block shown in FIGS. 1-3;
FIG. 6 is a cross-sectional view of the block of FIG. 5 along lines 6-6;
FIG. 7 is a cross-sectional view of the cutting block of FIG. 5 along lines 7-7; and
FIG. 8 is a view of the cutting block of the present invention with an optical tracker having a blade mounted in the cutting slot of the block.

### DETAILED DESCRIPTION OF THE PREFERRED EMBODIMENT

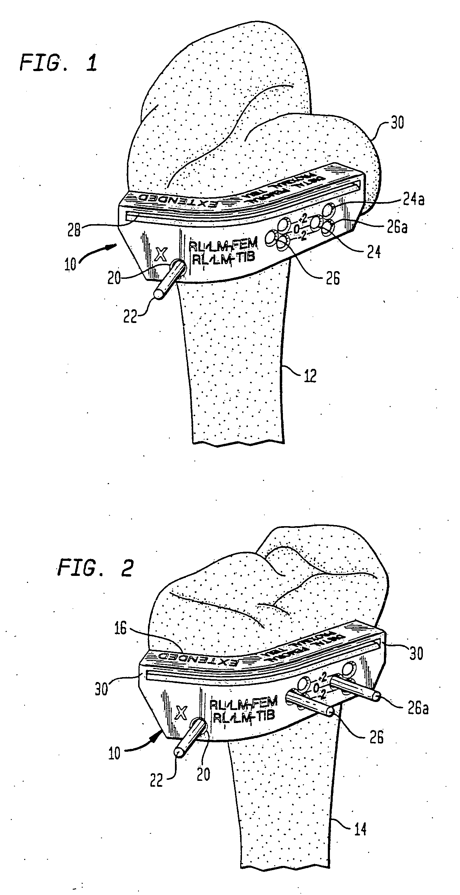

Referring to FIGS. 1-3, there is shown a cutting block generally denoted as 10 mounted on a femur 12. The cutting block 10 could also be shown on a tibia. Cutting block 10 has a curved inner surface 16 adapted to conform to the anterior-lateral or anterior-medial sides of the femur or tibia 12, 14 respectively. The cutting block, in the preferred embodiment, has a pin hole 20 adapted to receive a bone cross-locking pin 22 and a plurality of additional pin holes 24 located on an opposite end of block 10. In the preferred embodiment, there are six holes 24 spaced in the proximal-distal direction by 2 mm. Holes 24 are adapted to receive at least one or more pins 26, 26a which serve to lock or fix the block to the bone and prevent angular rotation. Cutting block 10 also includes a saw blade guide slot 28.

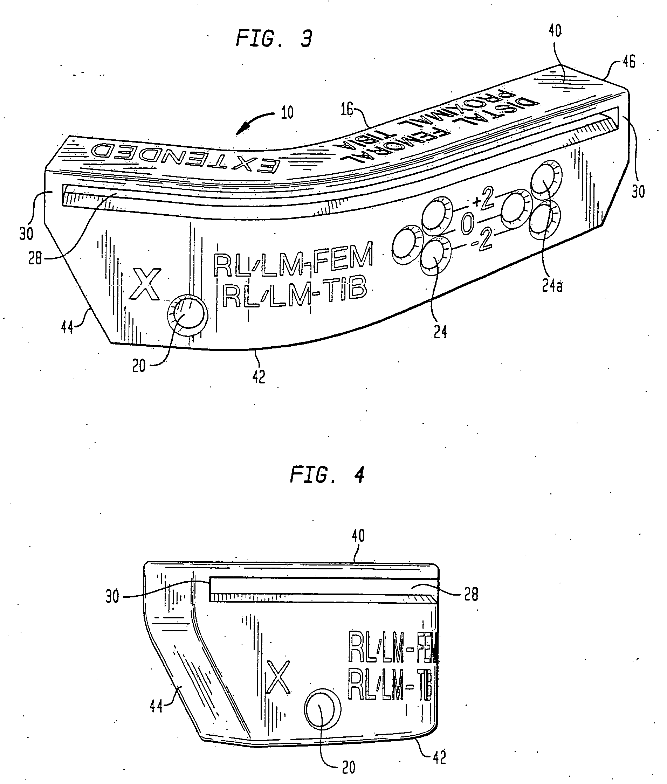

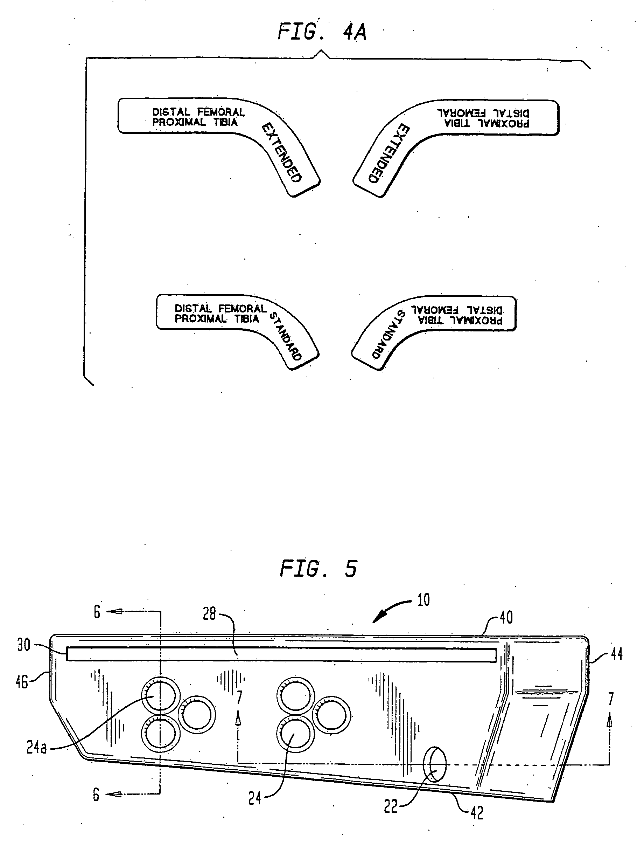

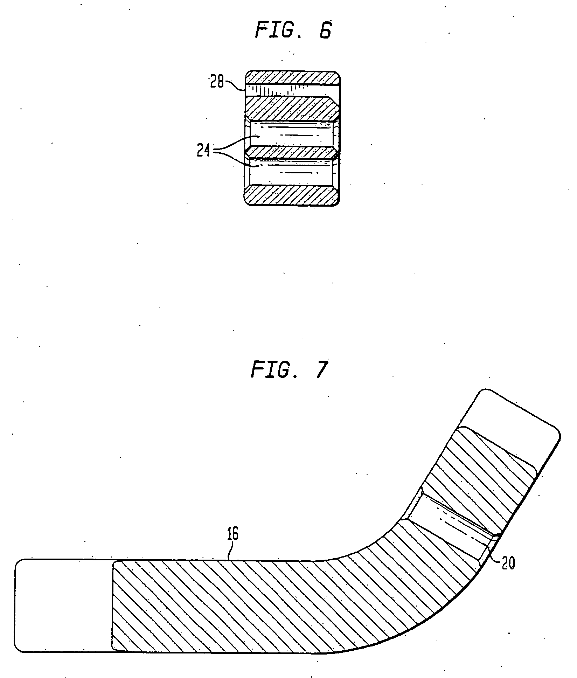

Referring to FIGS. 4-7, there is shown side and rear elevation views as well as cross-sectional views of the blocks shown in FIGS. 1-3. Block 10 includes a joint facing surface 40 which, in the femur, faces distally and in the tibia faces proximally. In the preferred embodiment, surface 40 is formed, at least in part, by a cross member which forms the top of slot 28. It is also possible to eliminate slot 28 and utilize only the top surface 40 of the block to guide a saw blade, such as an oscillating saw blade typically used to form bone cuts and knee resections.

As can be best seen in FIG. 5, the bottom surface 42 of block 10 may be tapered in direction away from surface 40. Thus, the block may be wider at end 44 thereof than at end 46. Thus, the distance between surface 40 and 42 at the end of block 10 at end 44 would be wider and end 46 narrower. In addition, the ends 44, 46 may be tapered inwardly on moving from surface 40 to surface 42 as shown in FIGS. 4 and 5.

In the preferred embodiment, guide slot 28 extends almost all the way across the length of surface 40 of block 10 and terminates in end wall surfaces 30. It is also possible to use a cylindrical pin at each end of slot 28 rather than a flat wall 30. A pin would allow an oscillatory saw blade to pivot around the pin to make the various cuts.

In use, the surgeon preferably mounts the optical tracker on the cutting block with block 10 detached from the bone. The computer-aided navigation system then allows the surgeon to free-hand guide the cutting block to the correct proximal-distal alignment and the correct angular alignment in either the varus-valgus or flexion-extension angular orientations depending on whether the surgeon is resecting the femur or the tibia. The block is also aligned in the correct internal-external rotation orientation in a freehand manner.

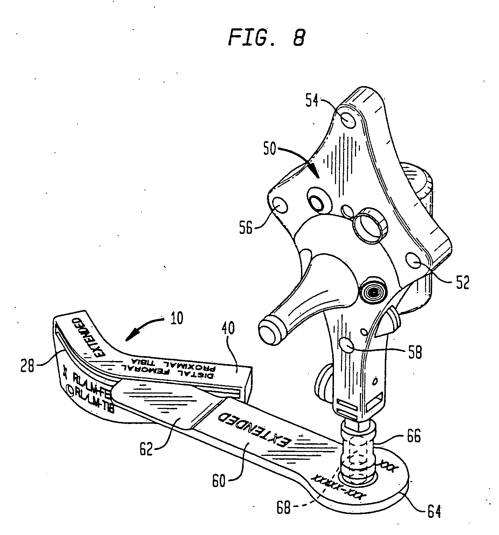

FIG. 8 shows a typical optical tracker 50 including, in the preferred embodiment, four light emitting diodes 52, 54, 56, 58. In the preferred embodiment, optical tracker 50 is removably coupled to a plate 60 which has a leading portion 62 designed to fit within slot 28. Plate 60 includes a second end 64 which includes a coupling element 66 which engages a mating coupling portion 68 on tracker 50. Alternately, the coupling 66 could be mounted directly on block 10 itself so that tracker 50 could be directly mounted to the block.

During use, the surgeon aligns block 10 free hand by inserting end 62 of plate 60 into slot 28 or attaching the tracker directly to the block and utilizing the image produced by the computer-optical navigation system, which image may include the mechanical axes and other alignment features generated by the navigation system based on the patient's anatomy, allowing the surgeon to locate the position of block 10 with respect to either the proximal tibia or distal femur and insert first pin, preferably 26a. Likewise, the movement of the cutting blocks and the setting of pin 22 in hole 20 would also be accomplished with the tracker 50 mounted on plate 60 with the assembly engaging cutting slot 28.

In both the tibia and femur, the surgeon uses the navigation system to first set the proximal-distal location freehand and then, in the femur, tilts the cutting block to set the correct varus-valgus alignment and then inserts a pin 26a into the femur through one of the six holes, preferably hole 24a of FIG. 1. Once pin 26a is set in the femur the surgeon sets the correct flexion-extension and then sets an additional pin 26 and finally sets pin 22 in hole 20.

In the tibia, the surgeon sets the proximal-distal location and the flexion-extension angle (slope) using the navigation system and inserts a pin 26a. The varus-valgus angle is then set and an additional pin 26 is inserted and finally pin 22 inserted in hole 20. In both the femur and tibia additional extra pins 26 are used after pin 22 is inserted for further fixation.

When using a computer-aided navigation system having a CRT for a series of displaying navigation screens changed with a handheld remote control for the resections, the navigation screen is advanced to the distal femoral resection screen using the hand held navigation control. The distal femoral resection requires the surgeon to position cutting block 10 in relation to the three axes of freedom - varus-valgus, flexion-extension and distal resection depth. In the preferred method, an optical tracker 50 is attached to a plate or blade which in turn is placed into slot 28 of the cutting block. The cutting block/tracker construction (see FIG. 8) is now an "active tool" whose virtual position can be monitored on the computer navigation screen.

The surgeon first places cutting block 10 against the medial or lateral surface of the distal femur. When positioning the cutting block, the long flat surface of the block should be against the medial or lateral side of the distal femur, while the shorter curved portion of the block wraps over the anterior part of the femoral condyle. Then in a similar method to arthroscopy, the surgeon watches the navigation screen as he moves the block into the desired position with one hand, leaving the second hand free to hold the pin driver. The resection depth (proximal-distal depth) is achieved by moving the block in a proximal-distal direction. Flexion-extension of the block is achieved by rotating the block in the appropriate flexion-extension direction. Lastly, varus-valgus positioning of the cutting block is achieved by tilting the block in a medial or lateral direction relative to the long axis of the femur. As each one of the movement is linked to the other, it is best to get each one correct to within 2 degrees with coarse hand movements, before adjusting final position with finer hand movements.

Once cutting block 10 is positioned to within a degree of the final position one pin 26 is inserted. This gives the block some stability against the side of the femur. The position of block 10 can still be altered by rotating around the single pin 26a to gain correct flexion-extension. The second fixation pin 26 is then inserted into the bone. Final fixation of the block is achieved by inserting pin 22 into cross pin hole 20.

In the preferred method, a blunt curved retractor is placed under the patella/patella tendon, with its top in the lateral gutter of the knee. This retractor acts as a tissue protector rather than a true retractor, as it separates the quads/patella mechanism from the saw blade. With cutting block 10 mounted on the medial side and with the knee in flexion, an oscillating saw is then used to cut from a medial to lateral direction through the flat surface of slot 28 of the block. The curved portion of the block can be used to cut the femoral condyles in an antero-posterior direction. In the preferred procedure, the resected part of the condyle is removed and the plate with tracker attached of FIG. 8 is placed on the distal cut surface to verify the depth and accuracy of the cut. This is then recorded on the femoral cut verification screen. When block 10 is placed on the lateral side of the distal femur a similar procedure is used but the saw is used to cut in a lateral to medial direction.

The navigation screen is next advanced to the proximal tibia resection using the handheld navigation working tool. The same cutting block is used, as for the distal femoral resection, with the resection plate with tracker attached placed on the captured slot. However, now the longer side of block 10 is placed adjacent the anterior tibia. A similar freehand technique of cutting block placement is also used, as for the distal femur. The cutting block is first placed into the wound and medial soft tissue envelope, created during the initial dissection. The surgeon then orients block 10 so the correct depth, flexion-extension angles (slope) and varus-valgus angles are achieved. The depth is achieved by proximal/distal movement of the block, while flexion-extension is achieved by tilting the block in an anterior-posterior direction about the long 'axis of the tibia. The desired varus-valgus angle for resection is achieved by rotating the block about the first pin. The virtual movements of the block can be monitored in real time on the navigation screen.

In a similar fashion to the distal femoral resection, the varus-valgus, depth and slope of the block are set to within 2 degrees with coarse hand movements, before adjusting final position with finer hand movements. Once cutting block 10 is positioned to within a degree of the final position one pin 26 is inserted. This gives the block some stability against the front of the tibia. The blocks position can still be altered by rotating around the single pin to gain correct varus-valgus angle. A second fixation pin 26 is then inserted and the position of the block checked on the navigation screen. If satisfactory cross pin 22 is inserted.

In the preferred procedure, a retractor is placed under the patella ligament and another placed to protect the medial collateral ligament. With the knee at 90 degrees of flexion, a saw blade is then introduced into slot 28 and the medial part of the tibial plateau is cut through the anterior portion of cutting block 10. The saw blade is then turned obliquely through the curved portion of the cutting block and the anterior portion of the lateral 'tibial plateau is cut (assuming the shorter curved section has been placed on the medial tibia). Next a curved retractor is placed behind the central tibial plateau to protect the posterior cruciate ligament and the central and posterior parts of the proximal tibia are cut. Finally a malleable retractor is inserted between the lateral collateral ligament and lateral tibia plateau, and the posterior-lateral tibial plateau is cut. This latter cut needs to be performed carefully to avoid damage to the lateral collateral ligament.

The knee is then placed into extension, where the previously resected distal femur provides space. An osteotome is used to free the cut proximal plateau and graspers are then utilized to remove the resected piece of bone. Soft tissue attachment to the resected bone is removed from medial side then the posterior aspect and finally the lateral side. The resected piece of bone is then removed. Once the resected bone has been removed the resection level can be measured with the plate with tracker attached of FIG. 8 to check its accuracy. The final cut can then be recorded on the tibial cut verification screen.

The cutting block 10 is preferably supplied in kits of four blocks which can be used interchangeably on the femur and the tibia. As shown in FIG. 4a, two of the blocks are of a standard length and two of the blocks are of an extended length. Each of the two standard blocks or each of the two extended blocks can be used to resect the femur and the tibia from either the medial or lateral sides of the femur or tibia. Thus, a single block 10 can be used to resected the left femur from the lateral side or the left tibia from the lateral side. In this case, the longer leg is positioned on the lateral side of the femur or on the anterior of the tibia. In the case of the femur, the cutting slot is positioned distally and in the case of the tibia, it is positioned proximally. On the right femur, this same block can be used in a similar manner to resect the medial side of the right femur or the medial side of the right tibia. The second standard block of the kit is designed to be used on the medial side of the left femur or on the medial side of the left tibia. This block likewise can be used to resect the right femur or right tibia from the lateral side. Thus, two blocks of standard length can be used to make proximal tibia and distal femur cuts from eight different positions.

Likewise, two blocks are supplied with an extended length for making the same proximal tibial cuts and distal femoral cuts on larger knees, again from eight different positions. Again, the shape of the block conforms to the medial and lateral quadrants of the left and right proximal tibia and left and right distal femur.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A kit of bone cutting blocks for resecting the distal femur and proximal tibia comprising:
a first block conforming in shape to the anterior-medial quadrant of the distal left femur and proximal left tibia and the anterior-lateral quadrant of the distal right femur and proximal right tibia; and
a second block conforming in shape to the anterior-lateral quadrant of the distal left femur and proximal left tibia and the anterior-medial quadrant of the distal right femur and proximal right tibia.

2. The kit as claimed in Claim 1 wherein the kit further comprises:
a third block conforming in shape to the anterior-medial quadrant of the distal left femur and proximal left tibia and the anterior-lateral quadrant of the distal right femur and proximal right tibia; and
a fourth block conforming in shape to the anterior-lateral quadrant of the distal left femur and proximal left tibia and the anterior-medial quadrant of the distal right femur and proximal right tibia.

3. The kit as claimed in Claim 2 wherein said anterior-medial and anterior-lateral quadrants each of said blocks contain one through hole (20) for securing a bone pin.

4. The kit as claimed in Claim 3 wherein each of said blocks have an anterior portion extending from said anterior-lateral and anterior-medial quadrants having a plurality of holes (24) for receiving bone pins (26).

5. The kit as claimed in Claim 4 wherein the anterior portion of said third and fourth blocks extend further than the anterior portion of said first and second blocks.

6. The kit as claimed in Claim 4 wherein there are six holes (24) spaced by 2mm in the proximal-distal directions when mounted on the femur or tibia.

7. The kit as claimed in any preceding claim wherein said blocks have a saw guide surface thereon.

8. The kit as claimed in Claim 7 wherein said saw guide surface is a slot (28) for receiving a saw blade.

9. The kit as claimed in any preceding claim further comprising a tracker having light emitting diodes thereon.

10. The kit as claimed in any preceding claim further comprising a plurality ofbone pins.
